# EUROPEAN PATENT SPECIFICATION

(11) **EP 1 369 120 B1**
(45) Date of publication and mention of the grant of the patent: **14.02.2007**
(21) Application number: 03012517.3
(22) Date of filing: 02.06.2003
(51) Int. Cl.: A61K 33/00, A61K 33/24, A61P 31/14

(54) **Treatment of flavivirus infection**
Behandlung von Flavivirusinfizierungen
Traitement de l'infection causé par les flavivirus

(30) Priority: 03.06.2002 US 385031 P
(43) Date of publication of application: 10.12.2003
(73) Proprietor: National Health Research Institutes, Taipei, 11529 (TW)
(72) Inventor: Hsu, Tsu-An, Ming-Sheng E.Road, Taipei (TW); Tsai, Yuan-Chin, Taipei (TW); Hwang, Der-Ren, Keelung, Taiwan (TW)
(74) Representative: Brandenburg, Thomas

(56) References cited:
- EP-A- 1 108 359
- WO-A-95/11033
- WO-A-02/096412
- US-A1- 2003 039 702

## Description

### BACKGROUND

Flaviviridae is a family of enveloped, positive single-stranded RNA viruses. It includes hepatitis C virus ("HCV"), GB virus, dengue virus, Japanese encephalitis virus, St. Louis encephalitis virus, West Nile virus, and yellow fever virus. Flaviviridae infection is a worldwide health problem.

For example, chronic HCV infection is estimated to affect 170 million people (WHO (1997) *Wkly Epidemiol Rec.* 72:341-344). Patients with liver damage resulting from HCV infection may develop chronic liver diseases, such as cirrhosis and hepatocellular carcinoma (Bartenschlager & Lohmann (2000) *J Gen Virol*. 81:1631-1648). HCV infection has been treated with a combination of interferon α and ribavirin (1-β-D-ribofuranosyl-1,2,4-triazole-3-carboxamide). See, e.g., Foster & Thomas (2000) *Baillieres Best Pract Res Clin Gastroenterol.* 14:255-64. The most current therapy involves using a modified interferon α, peginterferon α-2b, which has superior pharmacokinetic properties. Glue *et al*. (2000) *The Hepatitis C Intervention Therapy Group. Hepatology* 32: 647-53; and Lindsay *et al*. (2001) *Hepatology* 34:395-403. However, only a limited number of patients are cured after treatment with interferon α and ribavirin (McHutchison *et al*. (1998) *N Engl J Med*. 339:1485-92). Other drawbacks to this therapy include: (i) significant side effects; (ii) high cost; and (iii) poor responsiveness to HCV1b, the most common genotype in the United States.

WO9511033 discloses pharmaceutical compositions containing polyoxometallates and pharmaceutically acceptable derivatives thereof. The use of such compounds or compositions in therapy for the treatment or prophylaxis of infections by viruses which are confirmed or probable members of the family Flaviviridae including Hepatitis C.

EPI 108359 relates to a biocidal material comprising, as an active ingredient, a compound represented by General Formula (Ag2O)a(A2O)b(BO)c(C2O3)d(SiO2)e(DO2)f(E2O5)g (wherein A represents at least one species selected from the group consisting of alkali metallic elements, Cu, H and ammonium; B is at least one species selected from the group consisting of Fe, Cu, Zn, alkali earth metallic element, Ni, Mn, Co, Cd, Hg and Au; C represents at least one species selected from the group consisting of Fe, Al, Mn, B, Co, Cr, V, Sc, Y, La, Ga, In, Sb and Bi; D represents at least one species selected from the group consisting of Ce, Mn, C, Hf and Os; E represents at least one member selected from P, Sb, V, Nb, Ta and Bi; the subscriptions respectively are numbers which satisfy 0<a, O<a+b< 15, 0</=c< 15, 0</=d<S, 0<e, 0<e+f<7.5, 0<g<3, 10</= a+b+c+3d+2(e+f)+5g</= 15).

WO02096412 disclose various embodiments directed to pentavalent antimonials, purified pentavalent antimonials, and pentavalent antimonials that are comprised of an antimonial portion and an organic moeity portion. Alternatively to agents and methods of screening agents which mimic the activity of pentavalent antimonials, particularly sodium stibogluconate and glucatime. An appropriate organic moeity may be selected based upon a desired interaction (e.g. steric action) with active site of a cellular component (e.g. a PTPase). In a further embodiment the active site includes a cysteine residue which may be impacted by the disclosed compositions.

US2003039702 disclose a compound for use as an antiviral drug, mainly containing salts of heteropolyanions consisting of a tungstoantimonate (III) vanadium-mixed metal oxide or related salts represented by formula [(XW9O33)2V3O3]p-, where p is a positive number between 9 and 12 and X is Sb, P. As or Bi and especially Sb. An antiviral drug having a broad spectrum of antiviral activity, high potent efficacy and low toxicity is provided.

Thus, there is a need to develop a therapy that can treat infection by HCV and other flaviviridae viruses more efficaciously.

### SUMMARY

This invention is based, in part, on use of arsenic-, antimony-, or bismuth-containing compounds for the preparation of therapeutic agents for treating flaviviridae infection.

A method for treating infection by a flaviviridae virus is further disclosed. The method includes administering to a subject in need thereof (including a subject identified as in need of such treatment) an effective amount of a compound of the formula:

A_{w}BₓC_{y}D_{z},

in which A is Li, Na, K, Rb, or Cs; B is As, Sb, or Bi; C is an oxygen or sulfur atom; D is a monodentate ligand, a bidentate ligand, or a tridentate ligand; w is 0, 1, 2, or 3; x is 1, 2, 3, or 4; y is 0, 1, 2, 3, 4, or 5; z is 0, 1, 2, 3, 4, or 5; and provided that at least one of y and z is not 0. Note that combinations of variables envisioned by this invention are only those that result in the formation of stable compounds. The term "stable", as used herein, refers to a compound which possesses stability sufficient to allow manufacture and which maintains the integrity of the compound for a sufficient period of time to be useful for the purposes detailed herein (e.g., therapeutic or prophylactic administration to a subject). Also note that As, Sb, and Bi delineated herein include bivalent, trivalent, and pentavalent forms.

A subset of the compounds covered by the formula described above is featured by that each of w and z is 0. In some embodiments, x is 2 and y is 3. Exemplary compounds include As₂O₃, Sb₂O₃, and Bi₂O₃. In other embodiments, x is 2 or 4, and y is 2, 4 or 5, or x is 1 and y is 1. Exemplary compounds include As₂O₅, AsS, As₂S₂, and As₄S₄. Another subset of the compounds is featured by that w is 1, 2, or 3 and z is 0. In some embodiments, w is 1 and y is 2. An exemplary compound is NaAsO₂. A third subset of the compounds is featured by that y is 0. In these compounds, D can be a monodentate ligand, B can be As or Sb, and w can be 0, 1, or 2. Exemplary compounds include AsI₃ and sodium stibogluconate (i.e., oxo(A)-gluconato-2,3,4-hydroxodisodic antimony (V) sodium salt). Commercially available sodium stibogluconate includes Pentostam^{®} (Glaxo Wellcome Inc., London, England).

To practice the method of this invention, arsenic-, antimony-, or bismuth-containing polymeric compounds, such as -O-As(O⁻)-O-As(O⁻)-O-As(O⁻)-, can be used. These compounds, upon administration to a subject, are hydrolyzed to compounds of formula (I).

As used herein, the term "monodentate ligand" refers to an ion that has one point at which it attaches to atom B, and may have a valence of one or two. Examples of a monodentate monovalent ligand include fluoro, chloro, bromo, iodo, cyano, hydroxyl, mercapto, alkoxyl, thioalkoxyl, nitrate, perchlorate, carboxylate, amide, trialkylsilyl, isocyanate, isothiocyanate, imidazole, phosphate, and alkyl- or aryl-sulfonate. Examples of a monodentate divalent ligand include oxygen, sulfur, selenide, telluride, and sulfate. The term "bidentate ligand" refers to a compound that possesses two points at which it attaches to atom B. Examples of bidentate ligands include tartrate and polyol-type ligand (e.g., meglumine or carbohydrates). The term "tridentate ligand" refers to a compound that possesses three points at which it attaches to atom B. Examples of tridentate ligands include citrate and polyol-type ligand (e.g., meglumine or carbohydrates). As used above, the term "alkyl" refers to a straight-chained or branched alkyl group containing I to 6 carbon atoms. Examples of alkyl groups include methyl, ethyl, n-propyl, isopropyl, tert-butyl, and n-pentyl. The term "alkoxyl" refers to a straight-chained or branched group containing 1 to 6 carbon atoms and an oxygen radical. The term "aryl" refers to a hydrocarbon ring system having at least one aromatic ring. Examples of aryl moieties include phenyl, naphthyl, and pyrenyl.

The method described above is treating infection by a flaviviridae virus, which can be hepatitis C virus, GB virus, dengue virus, Japanese encephalitis virus, St. Louis encephalitis virus, West Nile virus, or yellow fever virus. What is meant by "treating infection" includes use of a compound described above for preventing or treating cirrhosis, hepatocellular carcinoma, or other disease states secondary to flaviviridae infection. A subject in need of such treatment can be identified by a health care professional based on the results of any diagnostic method. The subject can be concurrently administered with an effective amount of ribavirin. It can also be concurrently administered with an effective amount of interferon α. Further, it can be concurrently administered with an effective amount of interferon α and ribavirin.

Ribavirin is 1-β-D-ribofuranosyl-1,2,4-triazole-3-carboximide, which can be chemically synthesized. Commercially available ribavirins include VILONA3 (ICN Pharmaceuticals, Canada). Examples of interferon α, which can be in unmodified or modified form, include interferon α-2a, interferon α-2b, and Pegylated interferon a (e.g., peginterferon α-2b). Commercially available interferons include ROFERON^{®}-A (Roche Pharmaceuticals, Nutley, NJ), INTRON A3 (Schering Corporation, Kenilworth, NJ), INFERGEN^{®} (Amgen, Thousand Oaks, CA), and WELLFERON3 (Glaxo Wellcome Inc., Research Triangle Park, NC). Alternatively, interferons can be prepared by a synthetic method (e.g., by using a peptide synthesizer) followed by proper folding *in vitro,* or by a recombinant method using *E. coli,* yeast, insect cells, plant cells, or mammalian cells. A combination of ribavirin and interferon α-2b is sold as REBETRON3 by Schering Corporation.

Both interferon α and ribavirin can be also in the form of a pharmaceutical acceptable salt. Such a salt, for example, can be formed between a negatively charged substituent (e.g., carboxylate) on interferon α or ribavirin and a cation. Suitable cations include, but are not limited to, sodium ion, potassium ion, magnesium ion, calcium ion, and an ammonium cation such as tetramethylammonium ion. Likewise, a positively charged substituent (e.g., amino) can form a salt with a negatively charged counterion. Suitable counterions include, but are not limited to, chloride, bromide, iodide, sulfate, nitrate, phosphate, or acetate.

As used herein, "concurrent administration" refers to administering a compound of the formula described above and ribavirin, administering the compound and interferon α, or administering the compound, interferon α, and ribavirin as a mixture, or administering each by itself either at the same time or at different times during a treatment period. Typically, ribavirin is administered orally with an amount of from 10 mg/day to about 50,000 mg/day. Interferon α is administered parenterally (e.g., intramusclar or intracutaneous) with an amount of from about 0.1 to about 50 million International Units per week (e.g., three times per week). The compound, on the other hand, can be administered orally, parenterally (e.g., intramuscular, intracutaneous, or intrathecal), by inhalation spray, or via an implanted reservoir.

In another aspect, this invention features a pharmaceutical composition that includes a compound described above and ribavirin, wherein each of the compound and the ribavirin is in an effective amount for treating flaviviridae infection. This invention also features a pharmaceutical composition that includes one or more of the aforementioned compounds and interferon α, optionally, ribavirin, wherein each of the compound, the interferon α, and the ribavirin, if present, is in an effective amount for treating flaviviridae infection. Also included within the scope of this invention is a pharmaceutical composition that includes a compound described above, wherein the compound has not been known to possess a pharmaceutical activity, and is in an effective amount for treating flaviviridae infection.

In a further aspect, this invention features a kit for treating infection by a flaviviridae virus. The kit includes one of the aforementioned compounds and ribavirin, wherein each of the compound and the ribavirin is in an effective amount for treating flaviviridae infection. Alternatively, the kit includes the compound and interferon α, or the compound, interferon α, and ribavirin, wherein each of the compound, the interferon α, and the ribavirin is in an effective amount for treating flaviviridae infection.

Other features, objects, and advantages of the invention will be apparent from the description and drawings, and from the claims.

### DESCRIPTION OF DRAWINGS

FIG. 1A depicts arsenic trioxide inhibiting replication of positive strand hepatitis C virus RNA.
FIG. 1B depicts arsenic trioxide inhibiting replication of negative strand hepatitis C virus RNA.
FIG. 2 depicts antimony trioxide (2 µM) inhibiting replication of hepatitis C virus RNA.

### DETAILED DESCRIPTION

This invention relates to use of a compound of the formula described in Summary for the preparation of a therapeutic agent for treating flaviviridae infection. The compound includes one or more arsenic, antimony, or bismuth atoms in one molecule. An exemplary compound is arsenic trioxide, which has been known as an antineoplastic agent. For example, arsenic trioxide was approved by the U.S. Food and Drug Administration (FDA) in the fall of 2000 for treating acute promyelocytic leukemia in patients not responding to other medication regimens. In 2001, the FDA also approved injection of arsenic trioxide for treating both chronic myeloid leukemia and acute myelocytic leukemia. Arsenic trioxide is commercially available as a drug. In another example, sodium stibogluconate (Pentostam^{®}) has been used virtually as the only substance in English-speaking East Africa for the treatment of kala-azar and post-kala-azardermal leishmaniasis. These arsenic-, antimony- or bismuth-containing compounds described above can be synthesized from inorganic chemicals. See, e.g., Handbook of Preparative Inorganic Chemistry Vol. 1, G. Brauer Ed, 2^{nd} ed., (1963) Academic Press, New York, p 600.

A method for treating flaviviridae infection is disclosed with one or more aforementioned compounds (or pharmaceutically acceptable derivatives thereof, or compositions including the compounds or derivatives thereof). The method includes administering to a subject in need thereof an effective amount of the compound. Optionally, the subject is concurrently administered with an effective amount of interferon α, or ribavirin, or interferon α and ribavirin. The term "treating" is defined as the application or administration of a composition including the compound described above, alone or in combination with interferon α, or ribavirin, or interferon α and ribavirin, to a subject, who has flaviviridae infection, a symptom of flaviviridae infection, a disease state secondary to flaviviridae infection, or a predisposition toward flaviviridae infection, with the purpose to cure, alleviate, relieve, remedy, prevent, or ameliorate the infection, the symptom of the infection, the disease state secondary to the infection, or the predisposition toward the infection. "An effective amount" is the amount of the aforementioned compound, interferon α, or ribavirin (the compound alone or in combination with interferon α or ribavirin, or in combination with both interferon α and ribavirin), which, upon administration to a subject in need thereof, is required to confer therapeutic effect on the subject. An effective amount of the compound can range from about 0.001 mg/Kg to about 1000 mg/Kg. An effective amount of interferon α can range from about 0.1 to about 50 million International Units (IU) per week. An effective amount of ribavirin can range from about 10 mg/day to about 50,000 mg/day. Effective doses vary, as recognized by those skilled in the art, depending on route of administration, excipient usage, and the possibility of co-usage with other agents for treating flaviviridae infection, such as ursodeoxycholic acid.

To practice the method of the present invention, the compound described above can be administered orally, parenterally, by inhalation spray, or via an implanted reservoir; and interferon α can be administered parenterally. The term "parenteral" as used herein includes subcutaneous, intracutaneous, intravenous, intramuscular, intraarticular, intraarterial, intrasynovial, intrasternal, intrathecal, intralesional and intracranial injection, as well as various infusion techniques, such as intrahepatic infusion.

A composition for oral administration can be any orally acceptable dosage form including tablets, capsules, emulsions and aqueous suspensions, dispersions and solutions. Commonly used carriers for tablets include lactose and corn starch. Lubricating agents, such as magnesium stearate, are also typically added to tablets. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

A sterile injectable composition (e.g., aqueous or oleaginous suspension) can be formulated according to techniques known in the art using suitable dispersing or wetting agents (such as, for example, Tween 80) and suspending agents. The sterile injectable preparation can also be a sterile injectable solution or suspension in a non-toxic parenterally acceptable diluent or solvent, for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (e.g., synthetic mono- or di-glycerides). Fatty acids, such as oleic acid and its glyceride derivatives, are useful in the preparation of injectables, as are natural pharmaceutically-acceptable oils, such as olive oil or castor oil, especially in their polyoxyethylated versions. These oil solutions or suspensions can also contain a long-chain alcohol diluent or dispersant, or carboxymethyl cellulose or similar dispersing agents.

An inhalation composition can be prepared according to techniques well known in the art of pharmaceutical formulation and can be prepared as solutions in saline, employing benzyl alcohol or other suitable preservatives, absorption promoters to enhance bioavailability, fluorocarbons, and/or other solubilizing or dispersing agents known in the art.

A pharmaceutically acceptable carrier is routinely used with the aforementioned compound or interferon α. Such a carrier is compatible with the active ingredient of the formulation and not deleterious to the subject to be treated. For example, solubilizing agents, such as cyclodextrins (which form specific, more soluble complexes with the compound), can be utilized as pharmaceutical excipients for delivery of the compound. A capsule for encasing the compound such as arsenic trioxide is also a carrier. Examples of other carriers include colloidal silicon dioxide, magnesium stearate, cellulose, sodium lauryl sulfate, and D&C Yellow # 10.

An *in vitro* assay can be used to evaluate the efficacy of the compound described above, or together with interferon α (in various forms), in inhibiting hepatitis C virus RNA replication. The efficacy and dosages can be further evaluated in animal studies with procedures well known in the art. Examples of efficacy-evaluating methods are provided below.

Without further elaboration, it is believed that the above disclosure has adequately enabled the present invention.

### Inhibition of HCV RNA Replication

Huh-7 cells containing HCV sub-genomic replicons (Ava.5) were provided by Apath, LLC. (St. Louis, MO). See, e.g., Blight *et al*. (2000) *Science* 290:1972-4. Cells were maintained in Dulbecco's modified Eagle's medium (DMEM) supplemented with 10% heat-inactivated fetal calf serum (FCS) in a humidified atmosphere containing 5% of CO_{2.} The medium also contained 1 mg/mL G418 (an aminoglycosidic antibiotic agent obtained from Promega, WI) as a selective pressure for the maintenance of HCV replicon's RNA replication. See, e.g., Lohmann *et al*. (2001) *J Virol.* 75:1437-49. Human interferon α (IFN-α), arsenic trioxide (As₂O₃), and antimony trioxide (Sb₂O₃) were obtained from Sigma (St. Louis, MO). Sodium arsenite (NaAsO₂), arsenic iodide, potassium antimony tartrate (PAT), and bismuth trioxide (Bi₂O₃) were also obtained from Sigma. IFN-α was stored at -20°C in small aliquots. A stock solution of As₂O₃ (10 mM) was prepared by dissolving As₂O₃ powder in 1 N NaOH. Stock solutions of Sb₂O₃ (10 mM) or Bi₂O₃ (10 mM) were prepared by dissolving Sb₂O₃ or Bi₂O₃ powders in HCl/H₂O (1:1). Stock solutions of PAT (50 mM) or NaAsO₂ (10 mM) were prepared by dissolving PAT or NaAsO₂ powders in H₂O.

Cells were treated with As₂O₃ (0 to 1 µM), with Sb₂O₃ at various concentrations (0 to 5 µM), or with As₂O₃ (0 to 100 nM) in combination with IFN-α (0 to 1 IU/mL). Then, cells were harvested after 24 and/or 72 hr of the treatment. Subsequently, total cellular RNA's were extracted and quantified as follows.

Total RNA of cells was extracted using RNeasy Mini Kit (QIAGEN). The concentration of total RNA was measured by absorption at 260 nm. Total RNA (1 µg) was then mixed with 50 picomoles of HCV specific reverse primer: A9412, 5'-GATGGC CTATTG GCCTGG AGGGG-3' (Lohmann *et al*. (2001) *J Virol*. 75:1437-49). RNA was added with RNase-free ddH₂O to a final volume of 10.5 µL and denatured for 10 min at 65°C. Reverse transcription (RT) reactions were carried out at 42°C for 1 hr using Expand-RT (Roche Biochemicals, Mannheim, Germany) in a total volume of 20 µL containing a previously denatured primer-RNA mixture, 1 mM each of deoxynucleotide triphosphates (dNTPs), 50 units of reverse transcriptase, and 20 units of RNase inhibitor (Promega, Madison, WI).

Quantitative PCR (Q-PCR) was carried out by LightCycler-DNA Master using SYBR Green I for real-time detection of double-stranded DNA (dsDNA) (Roche Diagnostics GmbH, Mannheim, Germany). The intensity of fluorescence was recorded versus PCR cycle numbers and the relative amounts of HCV RNA were calculated corresponding to the standard curve. The standard curve was derived from five serial dilutions of reference DNA, the corresponding cDNA of HCV RNA, under criteria recommended by the supplier.

The relative copy numbers HCV RNA and a house-keeping gene, glyceraldehyde-3-phosphate dehydrogenase (GADPH) or actin, were determined by Q-PCR utilizing the following primer sets:
HCV primers (positive strand):
   Forward: 5'-ACCAGAATACGACTTGGAGTTGATAA-3' (SEQ ID NO:1)
   Reverse: 5'-CACCCTTTTGCCAGATGCAT-3' (SEQ ID NO:2)
HCV primers (negative strand):
   Forward: 5'-CTCGACGTTGTCACTGA-3' (SEQ ID NO:3)
   Reverse: 5'-CCATCCGAGTACGTGC-3' (SEQ ID NO:4)
GADPH primers
   Forward primer: 5'-GAAGGTGAAGGTCGGAGTC-3' (SEQ ID NO:5)
   Reverse primer: 5',-GAAGATGGTGATGGGATTTC-3' (SEQ ID N0:6)
Actin primers:
   Forward primer: 5'-ATCCGCAAAGACCTGT-3' (SEQ ID NO:7)
   Reverse primer: 5'-GTCCGCCTAGAAGCAT-3' (SEQ ID NO:8)

PCR was performed as follows: (i) denaturing conditions: 95°C, 60 sec; (ii) amplification conditions: ramp to 95°C, 0 sec; 64°C, 10 sec; 72°C, 10 sec. The specificity of an amplification reaction was determined by performing a melting curve analysis with a temperature range from 65°C to 95°C.

HCV RNA copy numbers were divided by their corresponding GADPH copy numbers in the same sample for normalization. Effect of drug treatments on HCV RNA replication was represented by "relative copy number," which was obtained by comparing HCV RNA copy numbers with drug treatment to HCV RNA copy numbers without drug treatment. The latter was designated as 100%. All measurements were performed in duplicates or triplicates to reach statistical significance.

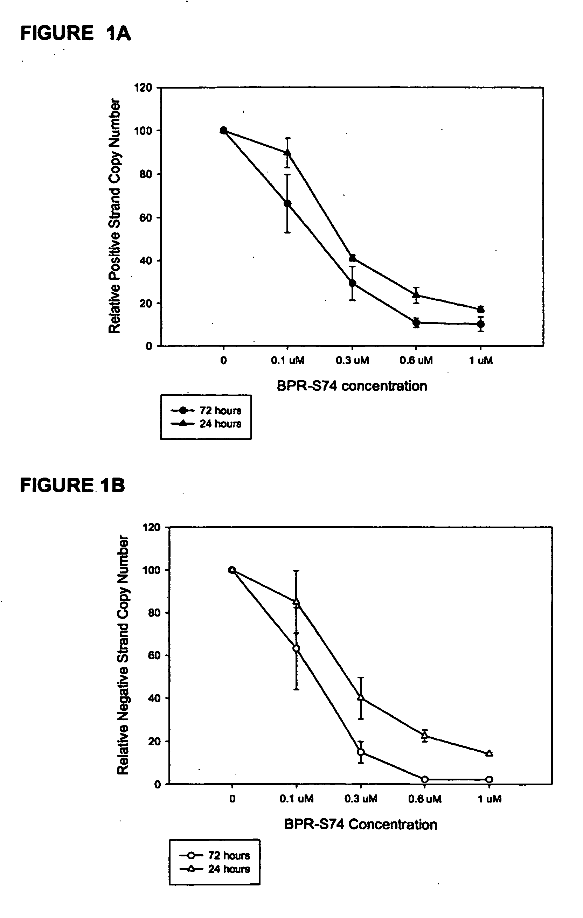

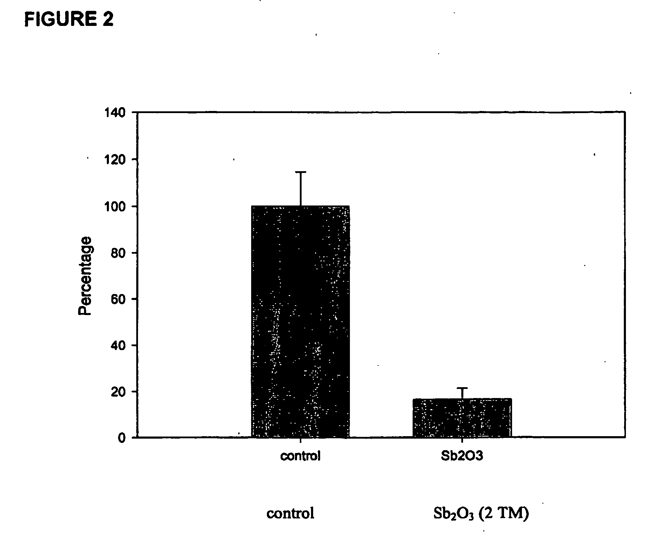

As₂O₃ inhibited replication of HCV RNA both positive strand (FIG. 1 A) and negative strand (FIG. 1B) in a dose-dependent manner. When replicon cells were exposed to 1 µM As₂O₃ for 24 hr, unexpectedly only 15.6% of the positive strand and 14.1% of the negative strand of HCV RNA were detected. It was also unexpected that when replicon cells were exposed to 1 µM As₂O₃ for 72 hr, only 6.1 % of positive strand RNA and 2.6% of negative strand HCV RNA were detected. Further, when replicon cells were exposed to 2 TM Sb₂O₃ for 24 hr, only 20% of HCV RNA were detected (FIG. 2).

In addition, when As₂O₃ was combined with IFN-α, additional inhibitory effect was achieved (Tables 1 and 2). In these experiments, replicon cells were treated with As₂O₃, alone or in combination with IFN-α, for 72 hr. In the absence of As₂O₃ and IFN-α, the relative HCV RNA levels were designated as 100%.

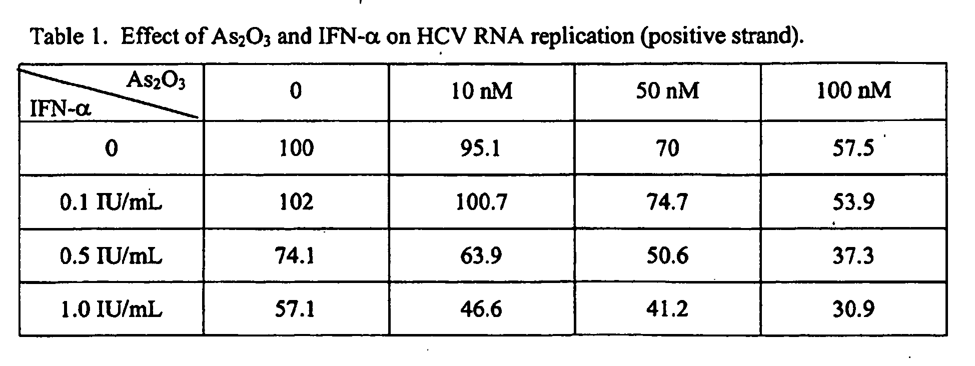

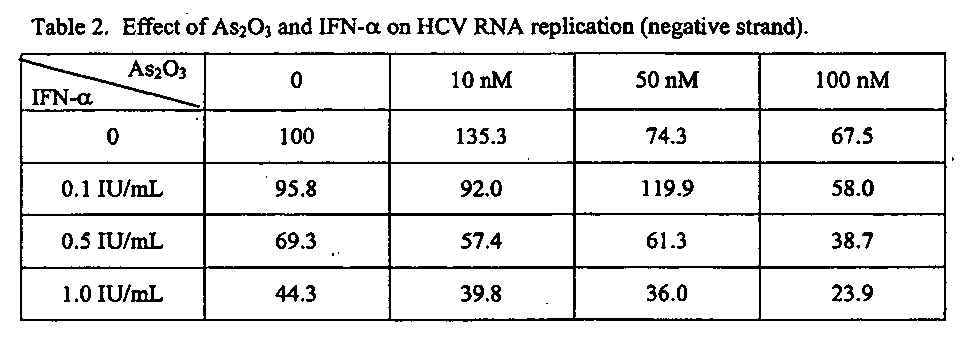

### Cell Growth Inhibition Assay

Huh-7 cells containing HCV sub-genomic replicons (Ava.5) were maintained in DMEM supplemented with 10% FCS in a humidified atmosphere containing 5% of CO_{2.} Cell viability was determined using 3-(4,5-dimethylthiazol-2-yl)-5-(3-carboxymethoxyphenyl)-2-(4-sulfophenyl)-2H-tetrazolium, inner salt (MTS), as described in Malich *et al*. (1997) *Toxicology* 124:179-92. MTS, in the presence of phenazine methosulfate (PMS), produced a water-soluble formazan product that had an absorbance maximum at 490-500 nm in phosphate-buffered saline (PBS). See, e.g., Cory *et al*. (1991) *Cancer Commun.* 3:207-12.

MTS and PMS, both in powdery form, were purchased from Sigma or Promega. MTS was dissolved in PBS (2 mg/mL) and PMS (0.38 mg/mL) was dissolved in ddH₂O as stock solutions. Cells cultured in a 96-well microtiter plate were treated with As₂O₃ at various concentrations. Prior to the assay, a 2 mL MTS/PMS solution containing MTS and PMS (20:1) was added to the cells with 8 mL of phenol red-free DMEM. The cell culture medium was then aspirated off, washed with PBS, and 100 µL of the MTS/PMS mixture was added into each well. After incubation at room temperature for 2 hr, optical densities of each sample were measured at OD₄₉₀ with a 96-well microtiter plate reader. Data were obtained with six repeats for each treatment with drugs.

Cyotoxicity of As₂O₃ on the HCV sub-genomic replicon cell line was determined by the just-described MTS assay. The ICso values (i.e., the concentration of As₂O₃ which causes 50% cell death after the cells are treated with As₂O₃) for 24 hr and 72 hr treatment with As₂O₃ were determined to be 8.5 µM and 4.8 µM, respectively. The EC₅₀ (i.e., the effective concentration of As₂O₃ which inhibits 50% of HCV RNA replication as determined by Q-PCR described above) and therapeutic index (TI = IC₅₀/EC₅₀) of As₂O₃ at different time intervals were shown in Table 3.

**Table 3. Effect of As₂O₃ on cell growth.**

| Measured By | EC₅₀ (µM) | Therapeutic Index |
|---|---|---|
| Positive strand (24 hr) | 0.26 | 8.5/0.26 = 32.7 |
| Positive strand (72 hr) | 0.19 | 4.8/0.19 = 25.3 |
| Negative strand (24 hr) | 0.27 | 8.5/0.27 = 31.5 |
| Negative strand (72 hr) | 0.16 | 4.8/0.16 = 30.0 |

Another HCV replication inhibition assay was performed using an Epstein-Barr virus-based extrachromosomal replication system as described in Yeh *et al*. (2001, *J. of Virology* 75:11017-11024). Results show that 300 nM As₂O₃ was able to inhibit the replication completely (data not shown).

By the same methods, EC₅₀ and IC₅₀ values for 24 hr treatment with sodium arsenite, arsenic iodide, antimony trioxide, potassium antimony tartrate, and bismuth trioxide were also determined using Ava5 cells (Table 4).

**Table 4. Anti-HCV effects.**

| Name | Formula | EC₅₀^{a} | IC₅₀^{b} | Note |
|---|---|---|---|---|
| Arsenic trioxide | As₂O₃ | 200 nM | 6 µM | |
| Sodium arsenite | NaAsO₂ | 250 nM | 10 µM | |
| Arsenic iodide | AsI₃ | 1 µM | 10 µM | |
| Antimony trioxide | Sb₂O₃ | 800 nM | 12.5 µM | |
| Potassium antimony tartrate (PAT) | C₈H₄O₁₂Sb₂K₂ | N.A.^{c} | 35 µM | 75 % inhibition of HCV replication at 5 µM of PAT treatment |
| Bismuth trioxide | Bi₂O₃ | N.A.^{c} | > 10 µM | 35 % inhibition of HCV replication at 2 µM of Bi₂O₃ treatment |

| | | | | |
|---|---|---|---|---|
| ^{a}ECₛₒ: Ava5 cells were harvested after 24 hours of drug treatment. Subsequently, total cellular RNA was extracted and quantified. The levels of HCV RNA (positive-strand) and the control mRNA of a housekeeping gene, GADPH or actin, were measured by Q-PCR. HCV RNA levels in each experiment were divided by respective GADPH and actin mRNA levels to obtain normalized HCV RNA levels. ^{b}IC₅₀: Cytotoxicity of compounds on Ava.5 cells was determined by MTS assay after treatment for 24 hours. ^{c}N.A.: not available. | | | | |

## Claims

1. Use of an effective amount of a compound having the following formula for the manufacture of a medicament for treating infection by a flaviviridae virus:
A_{W}B_{X}C_{Y}D_{Z}
wherein
A is Li, Na, K, Rb or Cs;
B is As, Sb or Bi;
C is an oxygen or sulfur atom;
D is a monodentate ligand, a bidentate ligand, or a tridentate ligand,
w is 0 1, 2 or 3;
x is 1, 2, 3, or 4;
y is 0, 1, 2, 3, 4, or 5;
z is 0, 1.2, 3, 4 or 5; and provided that at least one of y and z is not 0.

2. The use according to claim 1, wherein each of w and z is 0.

3. The use according to claim 2, wherein x is 2 and y is 3.

4. The use according to claim 3, wherein B is As.

5. The use according to claim 4, wherein the flaviviridae virus is hepatitis C virus.

6. The use according to claim 4, wherein the flaviviridae virus is GB virus, dengue virus, Japanese encephalitis virus, St. Louis encephalitis virus, West Nile virus, or yellow fever virus.

7. The use according to claim 4, wherein the medicament is for concurrent administration to a subject with an effective amount of ribavirin.

8. The use according to claim 4, wherein the medicament is for concurrent administration to a subject with an effective amount of interferon α.

9. The use according to claim 8, wherein the medicament is for concurrent administration to a subject with an ribavirin.

10. The use according to claim 3, wherein B is Sb.

11. The use according to claim 10. wherein the flaviviridae virus is hepatitis C virus.

12. The use according to claim 10, wherein the flaviviridae virus is GB virus, dengue virus, Japanese encephalitis virus, St. Louis encephalitis virus, West Nile virus, or yellow fever virus.

13. The use according to claim 10, wherein the medicament is for concurrent administration to a subject with an effective amount of ribavirin.

14. The use according to claim 10, wherein the medicament is for concurrent administration to a subject with an effective amount of interferon α.

15. The use according to claim 14, wherein the medicament is for concurrent administration to a subject with an effective amount of ribavirin.

16. The use according to claim 3, wherein B is Bi.

17. The use according to claim 16, wherein the flaviviridae virus is hepatitis C virus.

18. The use according to claim 16, wherein the flaviviridae virus is GB virus, dengue virus, Japanese encephalitis virus, St. Louis encephalitis virus, West Nile virus, or yellow fever virus.

19. The use according to claim 16, wherein the medicament is for concurrent administration to a subject with an effective amount of ribavirin.

20. The use according to claim 16, wherein the medicament is for concurrent administration to a subject with an effective amount of interferon α.

21. The use according to claim 20, wherein the medicament is for concurrent administration to a subject with an effective amount of ribavirin.

22. The use according to claim 1, wherein y is 0.

23. The use according to claim 22, wherein D is a monodentate ligand.

24. The use according to claim 23, wherein B is As or Sb, and w is 0, 1, or 2.

25. The use according to claim 24, wherein the flaviviridae virus is hepatitis C virus.

26. The use according to claim 24, wherein the flaviviridae virus is GB virus, dengue virus, Japanese encephalitis virus, St. Louis encephalitis virus, West Nile virus, or yellow fever virus.

27. The use according to claim 24, wherein the medicament is for concurrent administration to a subject with an effective amount of ribavirin.

28. The use according to claim 24, wherein the medicament is for concurrent administration to a subject with with an effective amount of interferon α.

29. The use according to claim 28. wherein the medicament is for concurrent administration to a subject with an effective amount of ribavirin.

30. The use according to claim 2, wherein each of x and y is 1.

31. The use according to claim 30, wherein the flaviviridae virus is hepatitis C virus, GB virus, dengue virus, Japanese encephalitis virus, St. Louis encephalitis virus, West Nile virus, or yellow fever virus.

32. The use according to claim 2, wherein x is 2 or 4, and y is 2, 4, or 5.

33. The use according to claim 32, wherein the flaviviridae virus is hepatitis C virus, GB virus, dengue virus, Japanese encephalitis virus, St. Louis encephalitis virus, West Nile virus, or yellow fever virus.

34. The use according to claim 1, wherein w is 1, 2, or 3 and z is 0.

35. The use according to claim 34, wherein w is 1 and y is 2.

36. The use according to claim 35, wherein B is As.

37. The use according to claim 36, wherein A is Na and C is an oxygen atom.

38. The use according to claim 37, wherein the flaviviridae virus is hepatitis C virus.

39. The use according to claim 37, wherein the flaviviridae virus is GB virus, dengue virus, Japanese encephalitis virus, St. Louis encephalitis virus, West Nile virus, or yellow fever virus.

40. The use according to claim 37, wherein the medicament is for concurrent administration to a subject with an effective amount of ribavirin.

41. The use according to claim 37, wherein the medicament is for concurrent administration to a subject with an effective amount of interferon α.

42. The use according to claim 41, wherein the medicament is for concurrent administration to a subject with an effective amount of ribavirin.

43. The use according to claim 1, wherein the flaviviridae virus is hepatitis C virus.

44. The use according to claim 1, wherein the flaviviridae virus is GB virus, dengue virus, Japanese encephalitis virus, St. Louis encephalitis virus. West Nile virus, or yellow fever virus.

45. The use according to claim 1, wherein the medicament is for concurrent administration to a subject with an effective amount of ribavirin.

46. The use according to claim 1, wherein the medicament is for concurrent administration to a subject with an effective amount of interferon α.

47. The use according to claim 46, wherein the medicament is for concurrent administration to a subject with an effective amount of ribavirin.

48. A pharmaceutical composition comprising:
a compound having the formula:
A_{w}BₓC_{y}D_{z},
in which A is Li, Na, K, Rb, or Cs; B is As, Sb, or Bi; C is an oxygen or sulfur atom; D is a monodentate ligand, a bidentate ligand, or a tridentate ligand; w is 0, 1, 2, or 3; x is 1, 2, 3, or 4; y is 0, 1, 2, 3, 4, or 5; z is 0, 1, 2, 3, 4, or 5; and provided that at least one of y and z is not 0; and
ribavirin;
wherein each of the ribavirin and the compound is in an effective amount for treating infection by a flaviviridae virus.

49. The pharmaceutical composition of claim 48, wherein the compound is As₂O₃, NaAsO₂, or Sb₂O₃.

50. A pharmaceutical composition comprising:
a compound having the formula:
A_{w}BₓC_{y}D_{z},
in which A is Li, Na, K, Rb, or Cs; B is As, Sb, or Bi; C is an oxygen or sulfur atom; D is a monodentate ligand, a bidentate ligand, or a tridentate ligand; w is 0, 1, 2, or 3; x is 1, 2, 3, or 4; y is 0, 1, 2, 3, 4, or 5; z is 0, 1, 2, 3, 4, or 5; and provided that at least one of y and z is not 0; and
interferon α;
wherein each of the interferon a and the compound is in an effective amount for treating infection by a flaviviridae virus.

51. The pharmaceutical composition of claim 50, wherein the compound is As₂O₃, NaAsO₂, or Sb₂O₃.

52. The pharmaceutical composition of claim 50, further comprising ribavirin, which is in an effective amount for treating the infection.

53. The pharmaceutical composition of claim 52, wherein the compound is As₂O₃, NaAsO₂, or Sb₂O₃.

54. A kit for treating infection by a flaviviridae virus, comprising:
a compound having the formula:
A_{w}BₓC_{y}D_{z},
in which A is Li, Na, K, Rb, or Cs; B is As, Sb, or Bi; C is an oxygen or sulfur atom; D is a monodentate ligand, a bidentate ligand, or a tridentate ligand; w is 0, 1, 2, or 3; x is 1, 2, 3, or 4; y is 0, 1, 2, 3, 4, or 5; z is 0, 1, 2, 3, 4, or 5; and provided that at least one of y and z is not 0; and
ribavirin;
wherein each of the compound and the ribavirin is in an effective amount for treating infection by a flaviviridae virus.

55. The kit of claim 54, wherein the compound is As₂O₃, NaAsO₂, or Sb₂O₃.

56. The kit of claim 54, wherein the flaviviridae virus is hepatitis C virus.

57. The kit of claim 54, wherein the flaviviridae virus is GB virus, dengue virus, Japanese encephalitis virus, St. Louis encephalitis virus, West Nile virus, or yellow fever virus.

58. A kit for treating infection by a flaviviridae virus, comprising:
a compound having the formula:
A_{w}BₓC_{y}D_{z},
in which A is Li, Na, K, Rb, or Cs; B is As, Sb, or Bi; C is an oxygen or sulfur atom; D is a monodentate ligand, a bidentate ligand, or a tridentate ligand; w is 0, 1, 2, or 3; x is 1, 2, 3, or 4; y is 0, 1, 2, 3, 4, or 5; z is 0, 1, 2, 3, 4, or 5; and provided that at least one of y and z is not 0; and
interferon α;
wherein each of the compound and the interferon α is in an effective amount for treating infection by a flaviviridae virus.

59. The kit of claim 58, wherein the compound is As₂O₃, NaAsO₂, or Sb₂O₃.

60. The kit of claim 58, wherein the flaviviridae virus is hepatitis C virus.

61. The kit of claim 58, wherein the flaviviridae virus is GB virus, dengue virus, Japanese encephalitis virus, St. Louis encephalitis virus, West Nile virus, or yellow fever virus.

62. The kit of claim 58, further comprising ribavirin, which is in an effective amount for treating the infection.

## Patentansprüche

1. Verwendung einer wirksamen Menge einer Verbindung aufweisend die folgende Formel zur Herstellung eines Medikamentes zur Behandlung der Infektion mit einem Flaviviridae-Virus:
A_{w}BₓC_{y}D_{z}
wobei
A für Li, Na, K, Rb oder Cs steht;
B für As, Sb oder Bi steht,
C für ein Sauerstoff-, oder ein Schwefelatom steht;
D für einen einzähnigen Liganden, einen zweizähnigen Liganden, oder einen dreizähnigen Liganden steht,
W für 0, 1, 2 oder 3 steht;
X für 1, 2, 3 oder 4 steht;
Y für 0, 1, 2, 3, 4 oder 5 steht;
Z für 0, 1, 2, 3, 4 oder 5 steht;
und
vorrausgesetzt dass mindestens eines von Y und Z nicht für 0 steht.

2. Die Verwendung nach Anspruch 1, wobei, jeweils W und Z für 0 stehen.

3. Die Verwendung nach Anspruch 2 wobei X für 2 und Y für 3 steht.

4. Die Verwendung nach Anspruch 3, wobei B für As steht.

5. Die Verwendung nach Anspruch 4, wobei der Flaviviridae-Virus der Hepatitis C Virus ist.

6. Die Verwendung nach Anspruch 4, wobei der Flaviviridae-Virus der GB-Virus der Dengue-Virus, der japanische Enzephalitis-Virus, der St. Louis Enzephalitis-Virus, der West-Nil-Virus oder der Gelbfieber-Virus ist.

7. Die Verwendung nach Anspruch 4, wobei das Medikament für die gleichzeitige Verabreichung an ein Subjekt zusammen mit einer wirksamen Menge von Ribavirin vorgesehen ist.

8. Die Verwendung nach Anspruch 4, wobei das Medikament für die gleichzeitige Verabreichung an ein Subjekt mit einer wirksamen Menge von Interferon alpha vorgesehen ist.

9. Die Verwendung nach Anspruch 8, wobei das Medikament für die gleichzeitige Verabreichung an ein Subjekt mit einer wirksamen Menge von Ribavirin vorgesehen ist.

10. Die Verwendung nach Anspruch 3, wobei B für Sb steht.

11. Die Verwendung nach Anspruch 10, wobei der Flaviviridae-Virus der Hepatitis C-Virus ist.

12. Die Verwendung nach Anspruch 10, wobei der Flaviviridae-Virus der GB-Virus, der Dengue-Virus, der japanische Enzephalitis-Virus, der St. Louis Enzephalitis-Virus, der West-Nil-Virus oder der Gelbfieber-Virus ist.

13. Die Verwendung nach Anspruch 10, wobei das Medikament für die gleichzeitige Verabreichung an ein Subjekt mit einer wirksamen Menge von Ribavirin vorgesehen ist.

14. Die Verwendung nach Anspruch 10, wobei das Medikament für die gleichzeitige Verabreichung an ein Subjekt mit einer wirksamen Menge von Interferon alpha vorgesehen ist.

15. Die Verwendung nach Anspruch 14, wobei das Medikament für die gleichzeitige Verabreichung an ein Subjekt mit einer wirksamen Menge von Ribavirin vorgesehen ist.

16. Die Verwendung nach Anspruch 3, wobei B für Bi steht.

17. Die Verwendung nach Anspruch 16, wobei der Flaviviridae-Virus der Hepatitis C-Virus ist.

18. Die Verwendung nach Anspruch 16, wobei der Flaviviridae-Virus, der GB-Virus, der Dengue-Virus, der japanische Enzephalitis-Virus, der St. Louis Enzephalitis-Virus, der West-Nil-Virus oder der Gelbfieber-Virus ist.

19. Die Verwendung nach Anspruch 16, wobei das Medikament für die gleichzeitige Verabreichung an ein Subjekt mit einer wirksamen Menge von Ribavirin vorgesehen ist.

20. Die Verwendung nach Anspruch 16, wobei das Medikament für die gleichzeitige Verabreichung an ein Subjekt mit einer wirksamen Menge von Interferon alpha vorgesehen ist.

21. Die Verwendung nach Anspruch 20, wobei das Medikament für die gleichzeitige Verabreichung an ein Subjekt mit einer wirksamen Menge von Ribavirin vorgesehen ist.

22. Die Verwendung nach Anspruch 1, wobei Y für 0 steht.

23. Die Verwendung nach Anspruch 22, wobei D für einen einzähnigen Liganden steht.

24. Die Verwendung nach Anspruch 23, wobei B für As oder Sb steht und W für 0, 1 oder 2 steht.

25. Die Verwendung nach Anspruch 24, wobei der Flaviviridae-Virus der Hepatitis C-Virus ist.

26. Die Verwendung nach Anspruch 24, wobei der Flaviviridae-Virus, das GB-Virus, der Dengue-Virus, der japanische Enzephalitis-Virus, der St. Louis Enzephalitis-Virus, der West-Nil-Virus oder der Gelbfieber-Virus ist.

27. Die Verwendung nach Anspruch 24, wobei das Medikament für die gleichzeitige Verabreichung an ein Subjekt mit einer wirksamen Menge von Ribavirin vorgesehen ist.

28. Die Verwendung nach Anspruch 24, wobei das Medikament für die gleichzeitige Verabreichung an ein Subjekt mit einer wirksamen Menge von Interferon alpha vorgesehen ist.

29. Die Verwendung nach Anspruch 28, wobei das Medikament für die gleichzeitige Verabreichung an ein Subjekt mit einer wirksamen Menge von Ribavirin vorgesehen ist.

30. Die Verwendung nach Anspruch 2, wobei X und Y jeweils 1 sind.

31. Die Verwendung nach Anspruch 30, wobei der Flaviviridae-Virus, der Hepatitis C-Virus, der GB-Virus, der Dengue-Virus, der japanische Enzephalitis-Virus, der St. Louis Enzephalitis-Virus, der West-Nil-Virus oder der Gelbfieber-Virus ist.

32. Die Verwendung nach Anspruch 2, wobei X für 2 oder 4 steht und Y für 2, 4 oder 5 steht.

33. Die Verwendung nach Anspruch 32, wobei der Flaviviridae-Virus, der Hepatitis C-Virus, der GB-Virus, der Dengue-Virus, der japanische Enzephalitis-Virus, der St. Louis Enzephalitis-Virus, der West-Nil-Virus, oder der Gelbfieber-Virus ist.

34. Die Verwendung nach Anspruch 1, wobei W für 1, 2 oder 3 und Z für 0 steht.

35. Die Verwendung nach Anspruch 34, wobei W für 1 und Y für 2 steht.

36. Die Verwendung nach Anspruch 35, wobei B für As steht.

37. Die Verwendung nach Anspruch 36 wobei A für Na und C für ein Sauerstoffatom steht.

38. Die Verwendung nach Anspruch 37, wobei der Flaviviridae-Virus der Hepatitis C-Virus ist.

39. Die Verwendung nach Anspruch 37, wobei der Flaviviridae-Virus, der GB-Virus, der Dengue-Virus, der japanische Enzephalitis-Virus, der St. Louis Enzephalitis-Virus, der West-Nil-Virus oder der Gelbfieber-Virus ist.

40. Die Verwendung nach Anspruch 37, wobei das Medikament für die gleichzeitige Verabreichung an ein Subjekt mit einer wirksamen Menge von Ribavirin vorgesehen ist.

41. Die Verwendung nach Anspruch 37, wobei das Medikament für die gleichzeitige Verabreichung an ein Subjekt mit einer wirksamen Menge von Interferon alpha vorgesehen ist.

42. Die Verwendung nach Anspruch 41, wobei das Medikament für die Verabreichung an ein Subjekt mit einer wirksamen Menge von Ribavirin vorgesehen ist.

43. Die Verwendung nach Anspruch 1, wobei das Flaviviridae-Virus das Hepatitis C-Virus ist.

44. Die Verwendung nach Anspruch 1, wobei der Flaviviridae-Virus, der GB-Virus, der Dengue-Virus, der japanische Enzephalitis-Virus, der St. Louis Enzephalitis-Virus, der West-Nil-Virus oder der Gelbfieber-Virus ist.

45. Die Verwendung nach Anspruch 1, wobei das Medikament zur gleichzeitigen Verabreichung an ein Subjekt mit einer wirksamen Menge von Ribavirin vorgesehen ist.

46. Die Verwendung nach Anspruch 1, wobei das Medikament zur gleichzeitigen Verabreichung an ein Subjekt mit einer wirksamen Menge von Interferon alpha vorgesehen ist.

47. Die Verwendung nach Anspruch 46, wobei das Medikament zur gleichzeitigen Verabreichung an ein Subjekt mit einer wirksamen Menge von Ribavirin vorgesehen ist.

48. Eine pharmazeutische Zusammensetzung umfassend:
eine Verbindung aufweisend die Formel:
A_{w}BₓC_{y}D_{z},
wobei A für Li, Na, K, Rb oder Cs steht; B für As, Sb, oder Bi steht; C für ein Sauerstoff- oder ein Schwefelatom steht; D für einen einzähnigen Liganden, einen zweizähnigen Liganden oder einen dreizähnigen Liganden steht; W für 0,1, 2 oder 3 steht; X für 1, 2, 3 oder 4 steht; Y für 0, 1, 2, 3, 4, oder 5 steht; Z für 0, 1, 2, 3, 4, oder 5 steht; und vorausgesetzt dass mindestens eines von Y oder Z nicht 0 ist; und
Ribavirin;
wobei jeweils Ribavirin und die Verbindung in einer wirksamen Menge zur Behandlung der Infektion durch einen Flaviviridae-Virus vorliegen.

49. Die pharmazeutische Zusammensetzung nach Anspruch 48, wobei die Verbindung aus As₂O₃, NaAsO₂, oder Sb₂O₃ besteht.

50. Eine pharmazeutische Zusammensetzung umfassend:
eine Verbindung mit der Formel:
A_{w}BₓC_{y}D_{z},
in der A für Li, Na, K, Rb oder Cs steht; B für As, Sb, oder Bi steht; C für ein Sauerstoff- oder ein Schwefelatom steht; D für einen einzähnigen Liganden, einen zweizähnigen Liganden oder einen dreizähnigen Liganden steht; W für 0, 1, 2 oder 3 steht; X für 1, 2, 3, oder 4 steht; Y für 0, 1, 2, 3, 4 oder 5 steht; Z für 0, 1, 2, 3, 4 oder 5 steht; und vorausgesetzt dass mindestens einer von Y oder Z nicht 0 ist; und
Interferon alpha;
wobei jeweils Interferon alpha und die Verbindung in einer wirksamen Menge zur Behandlung der Infektion durch einen Flavivirdae-Virus vorliegen.

51. Die pharmazeutische Zusammensetzung nach Anspruch 50, wobei die Verbindung As₂O₃, NaAsO₂ oder Sb₂O₃ ist.

52. Die pharmazeutische Zusammensetzung nach Anspruch 50 des Weiteren enthaltend Ribavirin, welches in einer wirksamen Menge zur Behandlung der Infektion vorliegt.

53. Die pharmazeutische Zusammensetzung nach Anspruch 52, wobei die Verbindung As₂O₃, NaAsO₂ oder Sb₂O₃ ist.

54. Eine Zusammenstellung zur Behandlung einer Infektion durch ein Flaviviridae-Virus, umfassend:
eine Verbindung aufweisend die Formel:
A_{w}BₓC_{y}D_{z},
wobei A für Li, Na, K, Rb oder Cs steht; B für As, Sb oder Bi steht; C für ein Sauerstoff- oder ein Schwefelatom steht; D für einen einzähnigen Liganden, einen zweizähnigen Liganden oder einen dreizähnigen Liganden steht; W für 0, 1, 2, oder 3 steht; X für 1, 2, 3 oder 4 steht; Y für 0, 1, 2, 3, 4 oder 5 steht; Z für 0, 1, 2, 3, 4 oder 5 steht; und vorausgesetzt dass mindestens einer von Y und Z nicht für 0 steht; und
Ribavirin;
wobei jeweils die Verbindung und das Ribavirin in wirksamer Menge zur Behandlung der Infektion durch einen Flaviviridae-Virus vorliegen.

55. Die Zusammenstellung nach Anspruch 54, wobei die Verbindung AS₂O₃, NaAsO₂, oder Sb₂O₃ ist.

56. Die Zusammenstellung nach Anspruch 54, wobei der Flaviviridae-Virus der Hepatitis C-Virus ist.

57. Die Zusammenstellung nach Anspruch 54, wobei der Flaviviridae-Virus, der GB-Virus, der Dengue-Virus, der japanische Enzephalitis-Virus, der St. Louis Enzephalitis-Virus, der Westnil-Virus oder der Gelbfieber Virus ist.

58. Eine Zusammenstellung zur Behandlung der Infektion durch einen Flaviviridae-Virus, umfassend:
eine Verbindung aufweisend die Formel:
A_{w}BₓC_{y}D_{z},
wobei A für Li, Na, K, Rb oder Cs steht; B für As, Sb, oder Bi steht; C für ein Sauerstoff- oder ein Schwefelatom steht; D für einen einzähnigen Liganden, einen zweizähnigen Liganden oder einen dreizähnigen Liganden steht; W für 0, 1, 2 oder 3 steht; X für 1, 2, 3 oder 4 steht; Y für 0, 1, 2, 3, 4 oder 5 steht; Z für 0, 1, 2, 3, 4 oder 5 steht; und vorausgesetzt dass mindestens einer von Y und Z nicht für 0 steht; und
Interferon alpha;
wobei jeweils die Verbindung und das Interferon alpha in einer wirksamen Menge zur Behandlung der Infektion durch einen Flaviviridae-Virus vorliegen.

59. Die Zusammenstellung nach Anspruch 58, worin die Verbindung As₂O₃, NaAsO₂, oder Sb₂O₃ ist.

60. Die Zusammenstellung nach Anspruch 58 wobei der Flaviviridae-Virus der Hepatitis C-Virus ist.

61. Die Zusammenstellung nach Anspruch 58, wobei der Flaviviridae-Virus, der GB-Virus, der Dengue-Virus, der japanische Enzephalitis-Virus, der St. Louis Enzephalitis-Virus, der West-Nil-Virus oder der Gelbfieber-Virus ist.

62. Die Zusammenstellung nach Anspruch 58, des weiteren umfassend Ribavirin, welches in wirksamer Menge zur Behandlung der Infektion vorliegt.

## Revendications

1. L'usage de la dose adéquate d'une composition, utilisée dans la préparation d'un médicament pour le traitement d'une infection causée par le virus flaviviridae, de formule suivante:
A_{w}BₓC_{y}D_{z}
dans laquelle
A correspond à Li, Na, K, Rb ou Cs;
B correspond à As, Sb ou Bi;
C est un atome d'oxygène ou de soufre;
D est un ligand monodental, un ligand bidental ou un ligand tridental,
w représente 0, 1, 2 ou 3;
x représente 1, 2, 3 ou 4;
y représente 0, 1, 2, 3, 4 ou 5;
z représente 0, 1, 2, 3, 4 ou 5; et
qu'il soit donné qu'au moins l'un des deux, y ou z, ne représente pas 0.

2. L'usage de la composition conformément à la revendication 1, selon laquelle w et z représente 0.

3. L'usage de la composition conformément à la revendication 2, selon laquelle x représente 2, et y représente 3.

4. L'usage de la composition conformément à la revendication 3, selon laquelle B est As.

5. L'usage de la composition conformément à la revendication 4, selon laquelle le virus flaviviridae est le virus de l'hépatite C.

6. L'usage de la composition conformément à la revendication 4, selon laquelle le virus flaviviridae est le virus GB, le virus de la dengue, le virus de l'encéphalite japonaise, le virus de l'encéphalite St. Louis, le virus West Nile ou le virus de la fièvre jaune.

7. L'usage de la composition conformément à la revendication 4, selon laquelle le médicament est prévu pour l'administration simultanée à un patient, avec une dose adéquate de ribavirin.

8. L'usage de la composition conformément à la revendication 4, selon laquelle le médicament est prévu pour l'administration simultanée à un patient, avec une dose adéquate d'interféron α.

9. L'usage de la composition conformément à la revendication 8, selon laquelle le médicament est prévu pour l'administration simultanée à un patient, avec une dose adéquate de ribavirin.

10. L'usage de la composition conformément à la revendication 3, selon laquelle B est Sb.

11. L'usage de la composition conformément à la revendication 10, selon laquelle le virus flaviviridae est le virus de l'hépatite C.

12. L'usage de la composition conformément à la revendication 10, selon laquelle le virus flaviviridae est le virus GB, le virus de la dengue, le virus de l'encéphalite japonaise, le virus de l'encéphalite St. Louis, le virus West Nile ou le virus de la fièvre jaune.

13. L'usage de la composition conformément à la revendication 10, selon laquelle le médicament est prévu pour l'administration simultanée à un patient, avec une dose adéquate de ribavirin.

14. L'usage de la composition conformément à la revendication 10, selon laquelle le médicament est prévu pour l'administration simultanée à un patient, avec une dose adéquate d'interféron α.

15. L'usage de la composition conformément à la revendication 14, selon laquelle le médicament est prévu pour l'administration simultanée à un patient, avec une dose adéquate de ribavirin.

16. L'usage de la composition conformément à la revendication 3, selon laquelle B est Bi.

17. L'usage de la composition conformément à la revendication 16, selon laquelle le virus flaviviridae est le virus de l'hépatite C.

18. L'usage de la composition conformément à la revendication 16, selon laquelle le virus flaviviridae est le virus GB, le virus de la dengue, le virus de l'encéphalite japonaise, le virus de l'encéphalite St. Louis, le virus West Nile ou le virus de la fièvre jaune.

19. L'usage de la composition conformément à la revendication 16, selon laquelle le médicament est prévu pour l'administration simultanée à un patient, avec une dose adéquate de ribavirin.

20. L'usage de la composition conformément à la revendication 16, selon laquelle le médicament est prévu pour l'administration simultanée à un patient, avec une dose adéquate d'interféron α.

21. L'usage de la composition conformément à la revendication 20, selon laquelle le médicament est prévu pour l'administration simultanée à un patient, avec une dose adéquate de ribavirin.

22. L'usage de la composition conformément à la revendication 1, selon laquelle y représente 0.

23. L'usage de la composition conformément à la revendication 22, selon laquelle D est un ligand monodental.

24. L'usage de la composition conformément à la revendication 23, selon laquelle B est As ou Sb et w représente 0, 1 ou 2.

25. L'usage de la composition conformément à la revendication 24, selon laquelle le virus flaviviridae est le virus de l'hépatite C.

26. L'usage de la composition conformément à la revendication 24, selon laquelle le virus flaviviridae est le virus GB, le virus de la dengue, le virus de l'encéphalite japonaise, le virus de l'encéphalite St. Louis, le virus West Nile ou le virus de la fièvre jaune.

27. L'usage de la composition conformément à la revendication 24, selon laquelle le médicament est prévu pour l'administration simultanée à un patient, avec une dose adéquate de ribavirin.

28. L'usage de la composition conformément à la revendication 24, selon laquelle le médicament est prévu pour l'administration simultanée à un patient, avec une dose adéquate d'interféron α.

29. L'usage de la composition conformément à la revendication 28, selon laquelle le médicament est prévu pour l'administration simultanée à un patient, avec une dose adéquate de ribavirin.

30. L'usage de la composition conformément à la revendication 2, selon laquelle x et y représentent chacun 1.

31. L'usage de la composition conformément à la revendication 30, selon laquelle le virus flaviviridae est le virus de l'hépatite C, le virus GB, le virus de la dengue, le virus de l'encéphalite japonaise, le virus de l'encéphalite St. Louis, le virus West Nile ou le virus de la fièvre jaune.

32. L'usage de la composition conformément à la revendication 2, selon laquelle x représente 2 ou 4, et y représente 2, 4 ou 5.

33. L'usage de la composition conformément à la revendication 32, selon laquelle le virus flaviviridae est le virus de l'hépatite C, le virus GB, le virus de la dengue, le virus de l'encéphalite japonaise, le virus de l'encéphalite St. Louis, le virus West Nile ou le virus de la fièvre jaune.

34. L'usage de la composition conformément à la revendication 1, selon laquelle w représente 1,2 ou 3, et z représente 0.

35. L'usage de la composition conformément à la revendication 34, selon laquelle w représente 1, et y représente 2.

36. L'usage de la composition conformément à la revendication 35, selon laquelle B est As.

37. L'usage de la composition conformément à la revendication 36, selon laquelle A est Na, et C est un atome d'oxygène.

38. L'usage de la composition conformément à la revendication 37, selon laquelle le virus flaviviridae est le virus de l'hépatite C.

39. L'usage de la composition conformément à la revendication 37, selon laquelle le virus flaviviridae est le virus GB, le virus de la dengue, le virus de l'encéphalite japonaise, le virus de l'encéphalite St. Louis, le virus West Nile ou le virus de la fièvre jaune.

40. L'usage de la composition conformément à la revendication 37, selon laquelle le médicament est prévu pour l'administration simultanée à un patient, avec une dose adéquate de ribavirin.

41. L'usage de la composition conformément à la revendication 37, selon laquelle le médicament est prévu pour l'administration simultanée à un patient, avec une dose adéquate d'interféron α.

42. L'usage de la composition conformément à la revendication 41, selon laquelle le médicament est prévu pour l'administration simultanée à un patient, avec une dose adéquate de ribavirin.

43. L'usage de la composition conformément à la revendication 1, selon laquelle le virus flaviviridae est le virus de l'hépatite C.

44. L'usage de la composition conformément à la revendication 1, selon laquelle le virus flaviviridae est le virus GB, le virus de la dengue, le virus de l'encéphalite japonaise, le virus de l'encéphalite St. Louis, le virus West Nile ou le virus de la fièvre jaune.

45. L'usage de la composition conformément à la revendication 1, selon laquelle le médicament est prévu pour l'administration simultanée à un patient, avec une dose adéquate de ribavirin.

46. L'usage de la composition conformément à la revendication 1, selon laquelle le médicament est prévu pour l'administration simultanée à un patient, avec une dose adéquate d'interféron α.

47. L'usage de la composition conformément à la revendication 46, selon laquelle le médicament est prévu pour l'administration simultanée à un patient, avec une dose adéquate de ribavirin.

48. Une composition pharmaceutique comprenant :
un composé de formule suivante :
A_{w}BₓC_{y} D_{z}
dans laquelle A est Li, Na, K, Rb ou Cs; B est As, Sb ou Bi; C est un atome d'oxygène ou de soufre; D est un ligand monodental, un ligand bidental ou un ligand tridental; w représente 0,1,2 ou 3; x représente 1, 2, 3 ou 4; y représente
0, 1, 2, 3, 4 ou 5; z représente 0, 1, 2, 3, 4 ou 5; et qu'il soit donné qu'au moins l'un des deux, y ou z, ne représente pas 0; et la ribavirin;
la ribavirin et le composé étant prévus chacun en dose adéquate pour le traitement d'une infection causée par le virus flaviviridae.

49. La composition pharmaceutique conformément à la revendication 48, dont le composé est As₂O₃, NaAsO₂ ou Sb₂O₃.

50. Une composition pharmaceutique comprenant:
un composé de formule suivante :
A_{w}B_{w}C_{y} D_{z}
dans laquelle A est Li, Na, K, Rb ou Cs; B est As, Sb ou Bi; C est un atome d'oxygène ou de soufre ; D est un ligand monodental, un ligand bidental ou un ligand tridental; w représente 0,1,2 ou 3; x représente 1, 2, 3 ou 4; y représente 0, 1, 2, 3, 4 ou 5; z représente 0, 1, 2, 3, 4 ou 5; et qu'il soit donné qu'au moins l'un des deux, y ou z, ne représente pas 0; et
l'interféron α;
l'interféron α et le composé étant prévus chacun en dose adéquate pour le traitement d'une infection causée par le virus flaviviridae.

51. La composition pharmaceutique conformément à la revendication 50, dont le composé est As₂O₃, NaAsO₂ ou Sb₂O₃.

52. La composition pharmaceutique conformément à la revendication 50, comprenant en outre de la ribavirin qui sert en dose adéquate au traitement de l'infection.

53. La composition pharmaceutique conformément à la revendication 52, dont le composé est As₂O₃, NaAsO₂ ou Sb₂O₃.

54. Une composition pour le traitement d'une infection causée par le virus flaviviridae, comprenant :
un composé de formule suivante:
A_{w}BₓC_{y} D_{z}
dans laquelle A est Li, Na, K, Rb ou Cs; B est As, Sb ou Bi; C est un atome d'oxygène ou de soufre; D est un ligand monodental, un ligand bidental ou un ligand tridental; w représente 0,1,2 ou 3; x représente 1, 2, 3 ou 4; y représente 0, 1, 2, 3, 4 ou 5; z représente 0, 1, 2, 3, 4 ou 5; et qu'il soit donné qu'au moins l'un des deux, y ou z, ne représente pas 0; et
la ribavirin;
la ribavirin et le composé étant prévus chacun en dose adéquate pour le traitement d'une infection causée par le virus flaviviridae.

55. La composition de la revendication 54, dont le composé est As₂O₃, NaAsO₂ ou Sb₂O₃.

56. La composition de la revendication 54, selon laquelle le virus flaviviridae est le virus de l'hépatite C.

57. La composition de la revendication 54, selon laquelle le virus flaviviridae est le virus GB, le virus de la dengue, le virus de l'encéphalite japonaise, le virus de l'encéphalite St. Louis, le virus West Nile ou le virus de la fièvre jaune.

58. Une composition pour le traitement d'une infection causée par le virus flaviviridae, comprenant :
un composé de formule suivante :
A_{w}BₓC_{y} D_{z}
dans laquelle A est Li, Na, K, Rb ou Cs; B est As, Sb ou Bi; C est un atome d'oxygène ou de soufre; D est un ligand monodental, un ligand bidental ou un ligand tridental; w représente 0,1,2 ou 3 ; x représente 1, 2, 3 ou 4 ; y représente 0, 1, 2, 3, 4 ou 5; z représente 0, 1, 2, 3, 4 ou 5; et qu'il soit donné qu'au moins l'un des deux, y ou z, ne représente pas 0; et
l'interféron α;
le composé et l'interféron α étant prévus chacun en dose adéquate pour le traitement d'une infection causée par le virus flaviviridae.

59. La composition de la revendication 58, dont le composé est As₂O₃, NaAsO₂ ou Sb₂O₃.

60. La composition de la revendication 58, selon laquelle le virus flaviviridae est le virus de l'hépatite C.

61. La composition de la revendication 58, selon laquelle le virus flaviviridae est le virus GB, le virus de la dengue, le virus de l'encéphalite japonaise, le virus de l'encéphalite St. Louis, le virus West Nile ou le virus de la fièvre jaune.

62. La composition de la revendication 58, comprenant en outre de la ribavirin qui sert en dose adéquate au traitement de l'infection.


### SEQUENCE LISTING

<110> National Health Research Institutes
<120> TREATMENT OF FLAVIVIRUS INFECTION
<130> 12563-014EP1
<150> US 60/385,031
   <151> 2002-06-03
<160> 9
<170> FastSEQ for Windows Version 4.0
<210> 1
   <211> 26
   <212> DNA
   <213> Artificial Sequence
<220>
   <223> Primer
<400> 1
   accagaatac gacttggagt tgataa 26
<210> 2
   <211> 20
   <212> DNA
   <213> Artificial Sequence
<220>
   <223> Primer
<400> 2
   cacccttttg ccagatgcat 20
<210> 3
   <211> 17
   <212> DNA
   <213> Artificial Sequence
<220>
   <223> Primer
<400> 3
   ctcgacgttg tcactga 17
<210> 4
   <211> 16
   <212> DNA
   <213> Artificial Sequence
<220>
   <223> Primer
<400> 4
   ccatccgagt acgtgc 16
<210> 5
   <211> 19
   <212> DNA
   <213> Artificial Sequence
<220>
   <223> Primer
<400> 5
   gaaggtgaag gtcggagtc 19
<210> 6
   <211> 20
   <212> DNA
   <213> Artificial Sequence
<220>
   <223> Primer
<400> 6
   gaagatggtg atgggatttc 20
<210> 7
   <211> 16
   <212> DNA
   <213> Artificial Sequence
<220>
   <223> Primer
<400> 7
   atccgcaaag acctgt 16
<210> 8
   <211> 16
   <212> DNA
   <213> Artificial Sequence
<220>
   <223> Primer
<400> 8
   gtccgcctag aagcat 16
<210> 9
   <211> 23
   <212> DNA
   <213> Artificial Sequence
<220>
   <223> Primer
<400> 9
   gatggcctat tggcctggag ggg 23

### SEQUENCE LISTING

<110> National Health Research Institutes
<120> TREATMENT OF FLAVIVIRUS INFECTION
<130> 12563-014EP1
<150> US 60/385,031
   <151> 2002-06-03
<160> 9
<170> Fast SEQ for Windows Version 4.0
<210> 1
   <211> 26
   <212> DNA
   <213> Artificial Sequence
<220>
   <223> Primer
<400> 1
   accagaatac gacttggagt tgataa 26
<210> 2
   <211> 20
   <212> DNA
   <213> Artificial Sequence
<220>
   <223> Primer
<400> 2
   cacccttttg ccagatgcat 20
<210> 3
   <211> 17
   <212> DNA
   <213> Artificial Sequence
<220>
   <223> Primer
<400> 3
   ctcgacgttg tcactga 17
<210> 4
   <211> 16
   <212> DNA
   <213> Artificial Sequence
<220>
   <223> Primer
<400> 4
   ccatccgagt acgtgc 16
<210> 5
   <211> 19
   <212> DNA
   <213> Artificial Sequence
<220>
   <223> Primer
<400> 5
   gaaggtgaag gtcggagtc 19
<210> 6
   <211> 20
   <212> DNA
   <213> Artificial Sequence
<220>
   <223> Primer
<400> 6
   gaagatggtg atgggatttc 20
<210> 7
   <211> 16
   <212> DNA
   <213> Artificial Sequence
<220>
   <223> Primer
<400> 7
   atccgcaaag acctgt 16
<210> 8
   <211> 16
   <212> DNA
   <213> Artificial Sequence
<220>
   <223> Primer
<400> 8
   gtccgcctag aagcat 16
<210> 9
   <211> 23
   <212> DNA
   <213> Artificial Sequence
<220>
   <223> Primer
<400> 9
   gatggcctat tggcctggag ggg 23